# EUROPÄISCHE PATENTANMELDUNG

(11) **EP 1 407 732 A1**
(43) Veröffentlichungstag der Anmeldung: **14.04.2004**
(21) Anmeldenummer: 03021873.9
(22) Anmeldetag: 27.09.2003
(51) Int. Cl.: A61F 9/00, A61K 9/00, A61M 35/00

(54) **Am Auge applizierbare Vorrichtung zur sicheren Dosierung systemischer und topischer Medikation**

(30) Priorität: 08.10.2002 DE 10246900
(71) Anmelder: Horstmann, Michael, Dr., 56564 Neuwied (DE)
(72) Erfinder: Horstmann, Michael, Dr., 56564 Neuwied (DE)

(57) **Zusammenfassung**

Folienartige Darreichungsform für pharmazeutische Wirkstoffe am Auge oder auf Schleimhäuten zur lokalen (topischen) oder systemischen Verwendung, welche leicht, bequem und regelmäßig für den Durchschnittsverbraucher applizierbar ist und eine hohe chemische und mikrobiologische Stabilität aufweist.

Die Darreichungsform umfaßt eine Applikationseinheit zur Anwendung von flächenförmigen wirkstoffhaltigen Darreichungsformen am Auge, bestehend aus der flächenförmigen Arzneiform selbst, einer Sollbruchstelle und einem Applikator.

## Beschreibung

Augentropfen sind eine seit Jahrhunderten übliche Arzneiform zur Therapie von lokalen Erkrankungen des Auges. In klassischer Vorgehensweise werden hier Lösungen der Arzneistoffe in wässrigen Medien zubereitet und nach Zugabe von verträglichkeitsverbessemden und stabilisierenden Stoffen abgefüllt. Die Anwendung beim Patienten erfolgt sowohl aus Mehrdosenbehältern, die nach Anbruch zwar nicht mehr steril, aber in den meisten Fällen hinlänglich keimarm bleiben, oder aber in Einzeldosenbehältern, für die in jedem Fall die Anwendung tatsächlich steriler (keimfreier) Lösungen gewährleistet ist.

Eine andere Form der Anwendung am Auge sind Augensalben. Diese werden insbesondere eingesetzt, wenn es auf eine langsame, über mehrere Stunden vorgesehene Arzneistoffzufuhr ankommt. Nachteile von Augensalben liegen vor allem in der mangelnden Transparenz der resultierenden Tränenflüssigkeit, aus welchen eine Anwendung am Tage in der Regel nicht in Frage kommt.

Neben Augentropfen und Augensalben sind in den letzten Jahrzehnten auch wasserunlösliche aber augenverträgliche, die Arzneistoffzufuhr kontrollierende Scheiben eingeführt worden (Ocusert, US Patent 4,135,514); diese Produkte sind in der Regel unlöslich. Seit langem sind allerdings wasserlösliche arzneistoffhaltige Gele, z.B. aus Glycerin-Gelatine bekannt, die sich zum Einlegen in das Auge eignen, um den Arzneistoff sofort freizusetzen (zitiert nach US Patent 3,960,150). In der neueren Zeit sind auch andere, weiter fortgeschrittene Formen löslicher Folienstücke zur Anwendung am Auge publiziert worden. Unter diesen ist z.B. US Patent 4,343,787 zu nennen, hier wird ein flacher Körper bestehend im wesentlichen aus Cellulosederivaten, Stärkederivaten, Polyethylenglykol, Polyphenylmethyläther, Polyethylenoxyd, Carboxyvinylpolymeren etc. erzeugt. Das flächenförmige Gebilde wird ins Auge eingelegt undlöst sich oder zerfällt unter Abgabe der Wirkstoffe nahezu sofort. Wie in EPO 0251680 (Iolab) beschrieben, können solche schnell zerfallenden Matrizes sich auch zur Abgabe bioerodierbarer Mikropartikel eignen, sodass eine zeitlich verzögerte Freigabe auch mit diesen schnellzerfallenden/schnellauflösenden Systemen möglich wird.

In US Patent 4,179,497 (Coven, Merck & Co.) wird Hydroxypropylcellulose als Trägerstoff empfohlen. Es wird auf den Vorteil der langsameren aber gleichmäßigen Freigabe des Wirkstoffes hingewiesen. Femer soll der Verlust des Wirkstoffes über den Nasen-Tränen-Gang soweit wie möglich reduziert werden. Das letztgenannte Phänomen ist einer der wesentlichen Gründe dafür, warum Augentropfen nur zu einem kleinen Teil (meist unter 10 % der Dosis) dem Auge selbst über die Resorption über die Comea zugeführt werden und der Hauptbestandteil der Flüssigkeit relativ schnell über den Nasen-Tränen-Gang letztendlich in den Nasen-Rachen-Raum gelangt und dort spätestens über den Gastrointestinaltrakt systemisch absorbiert wird. Dabei liegt der Vorteil von trockenen, flächenförmigen Darreichungsformen hier gegenüber Augentropfen sicherlich darin, dass sie das Tränenflüssigkeitsvolumen nicht so stark aufweiten und gleichzeitig die Viskosität der Tränenflüssigkeit anheben können.
Auch ist die Dosierungsgenauigkeit verbessert.

Allerdings muss die systemische Aufnahme von Augentropfen oder anderen im Auge applizierten Arzneiformen nicht unbedingt unerwünscht sein. Da es eine Reihe von Stoffen, insbesondere Polypeptide, gibt, für die es außer der intravenösen Zufuhr kaum akzeptable Resorptionsorte gibt, kann sich die Applikation über das Auge durchaus auch zu gezielten systemischen Medikation eignen (US Patent 5,182,258). Bereits für eine Reihe von Wirkstoffen ist die ausreichende systemische Verfügbarkeit über die Applikation über das Auge bereits beschrieben wie: Insulin, Glukagon, Enkephaline, Calcitonin und andere. Das bereits zitierte US Patent 5,182,258 nennt über solche Wirkstoffkandidaten hinaus die Möglichkeit der Erhöhung der Permeationsrate im Augen-Nasen-Gang z.B. durch Polyoxyethylen, Sorbitanmonooleat und andere Stoffe. Auch die gleichzeitige Verwendung von Peptidasehemmem ist erwähnt, wodurch die Wirkstoffe länger und intensiver zur Verfügung stehen.

Darougar et al. (US Patent 6,264,971) beschreiben genaue geometrische Maße von flächenförmigen Körpern zur Anwendung am Auge, die sinnvollerweise eine Länge von mindestens 8 mm und einen maximalen Durchmesser von nicht mehr als 1,9 mm aufweisen sollten.

Auch wenn flächenförmige leicht lösliche Arzneiformen am Auge bisher nicht breit eingeführt sind, erscheinen die Vorteile hinsichtlich der genauen Dosierbarkeit, der erreichbaren höheren Stabilität und der Vermeidung des Auswaschens durch die Tränenflüssigkeit doch sehr groß.

Gründe für die bisher mangelnde Akzeptanz und Marktdurchdringung der dem Stand der Technik entsprechenden flächenförmigen Produkte zur Anwendung am Auge hängen daher wohl mit der wenig üblichen und für den Durchschnittsverbraucher schwer durchführbaren Anwendung zusammen. Die Präparate müssen der Verpackung entnommen werden und es wird in der Regel erhebliche Probleme bereiten, ohne Verwendung von (haushaltsunüblichen) Sterilbestecken solche flächenförmigen Produkte mechanisch und hygienisch sicher in das Auge zu bringen.

Aufgabe dieser Erfindung ist daher die Bereitstellung einer folienartigen, flächenförmigen Darreichungsform für pharmazeutische Wirkstoffe am Auge oder auf Schleimhäuten zur lokalen (topischen) oder systemischen Verwendung, welche leicht, bequem und regelmäßig für den Durchschnittsverbraucher applizierbar ist und eine hohe chemische und mikrobiologische Stabilität aufweist.

Die Aufgabe wird gelöst durch eine Applikationseinheit zur Anwendung von flächenförmigen wirkstoffhaltigen Darreichungsformen am Auge, gekennzeichnet dadurch, dass diese Applikationseinheit aus der flächenförmigen Arzneiform, einer Sollbruchstelle und einem Applikator besteht.

Die Anwendung des Erfindungsgegenstandes durch den Anwender erfolgt nach Entnahme aus der Verpackung durch Anfassen am Applikatorteil, Öffnen des Auges, Auflagen des Teils mit der folienförmigen Arzneiform auf den Applikationsort am Auge, Abwarten der z.B. durch Feuchteeinfluss erfolgenden Abtrennung der Applikationsstelle und schließlich die Entfernung des Applikatorteils, der anschließend verworfen wird. Aus diese Weise wird eine direkte Berührung der folienförmigen Arzneiform mit den Fingern und demzufolge einer bakteriellen Kontamination vollständig vermieden. Da eine Einzelverpackung möglich ist, besteht -anders als bei Augentropfen in Sammelgefäßen- nicht die Gefahr einer Kontamination der Folgedosen.

Im Gegensatz zu einzeldosierten Arzneiformen wird sogar die Lagerung in flüssiger (wässriger) Form vermieden. Dieser Aspekt lässt nicht nur eine Arzneiformentwicklung ohne Zusatz von Stabilisatoren (z.B. bakteriziden Substanzen) zu, sondem es wird auch wegen der Möglichkeit des vollständigen Wasserausschlusses die Formulierung von Wirkstoffen möglich, die unter Einwirkung von Wasser nicht lagerstabil wären. Dieser Aspekt ist insbesondere für die Formulierung von Peptidwirkstoffen oder RNA/DNA/Plasmiden ein deutlicher Vorteil. Durch die klare geometrische Abgrenzung unter Verwendung der Sollbruchstelle ist weiterhin eine sehr genaue Dosierung der folienförmigen Arzneiform möglich. Diese ist mit Augentropfen, deren Dosierung von Zufälligkeiten der Oberflächenspannung, der Temperatur, und der Anwendungsweise abhängig ist, nicht möglich. Des Weiteren besteht anders als bei Augentropfen nicht die Gefahr des Überlaufens von zu viel wirkstoffhaltiger Flüssigkeit aus dem Auge, da die Arzneiform in kompakter Form am Augapfel oder im Bindehautsack verbleibt.

Die Erfindung wird im Folgenden näher beschreiben.

Die folienförmige Arzneiform weist vorzugsweise eine flache Kontur mit einer Dicke vorzugsweise zwischen 2 und 500 µm auf, in der Längsrichtung sind insbesondere Dimensionen zwischen 3 und 20 mm, in der Breite zwischen 1 und 12 mm bevorzugt. Die folienförmige Arzneiform wiegt üblicherweise zwischen 2 und 50 mg, kann diese Gewichtsbegrenzung aber auch deutlich über- und unterschreiten. Die Verwendung des Applikators erlaubt selbst die Applikation winzigster folienförmiger Arzneiformen (mit einer Masse von ca. 0,5-10 mg), was als weiterer vorteilhafter Aspekt hiermit hervorgehoben wird.
Als pharmazeutische Hilfsstoffe für den Aufbau der folienförmigen Arzneiform, aber auch des Applikators selbst, eigenen sich sämtliche pharmazeutisch üblichen Polymere, sofern sie schleimhautverträglich sind. Hier sind insbesondere hervorgehoben wasserlösliche und wasserquellbare Celluloseäther, Polyvinylalkohol, Agar-Agar, Gelatine, Carageenan, Stärkederivate (auch Cyclodextrin), Polyvinylpyrrolidon, Polyacrylsäure und ihre Salze, Polyamid, Pullulan und andere. Auch wenn anorganische Salze aus osmotischen Gründen (Gefahr der Reizung des Auges) zurückhaltend verwendet werden sollen, kann dies im Einzelfall sinnvoll sein, um die Verträglichkeit anzupassen. Hier sind insbesondere Natriumchlorid, Phosphate und Acetate der Alkali- und Erdalkalimetalle verwendbar. Auch die Verwendung von weiteren niedermolekularen Stoffen kann neben dem Wirkstoff selbst sinnvoll sein. Hier sind z.B. organische Verbindungen wie monomere und oligomere Zucker (Sorbit, Mannit, Xylit, Glukose, Lactose etc.) zu nennen, wie auch niedermolekulare organische Säuren wie Bemsteinsäure, Äpfelsäure usw., aber auch Substanzen wie Polyethylenglycol, Propylengylcol, Glycerin, etc., die weichmachenden Einfluss haben können. Die Verwendung von Füllstoffen kann aus Gründen der Adsorption von pharmazeutischen Wirkstoffen sinnvoll sein. Hier kann es sich um Carbonate, Phosphate und Silikate der Erdalkalimetalle handeln, oder Zinkoxyd oder Titandioxyd, um Siliziumoxyde aber auch andere Füllstoffe, sofern sie am Auge verträglich sind.

Bei den verwendeten Arzneistoffen kann es sich sowohl um lokal verwendete ophtalmische Wirkstoffe handeln wie Antibiotika (z.B. Tetracyclin, Bacitracin, Rifampicin, Gentamicin etc.) um antibakterielle Stoffe wie Sulfonamide, um antivirale Substanzen wie Idoxuridine, Axyclovir etc., Miotica und Cholinesterasehemmer wie Pilocarpin, Physostigmin, Impfstoffe, Immunstimulanzien, Corticosteroide wie Hydrocortison, Dexametason und andere, Mydriatica wie Atropin, Homatropin, Betablocker wie Timolol oder Betaxolol, und viele andere mehr.

Neben den vorstehenden Wirkstoffen, die im wesentlichen topische Wirkungen entfalten, sind auch Wirkstoffe zu nennen, die über die systemische Resorption aus dem Tränen-Nasen-Gang zur Resorption kommen und systemisch verfügbar sind, wie z.B. Insulin, Glukagon, Vasopressin, peptidische Impfstoffe, wie auch Impfstoffe auf Basis von Nukleinsäuren (DNA/RNA). Weiterhin geeignete peptidische Stoffe sind: Lipotropinderivate, Enkephaline, Endorphine, Thyreotropin Releasing Hormon, LH-RH, Oxytocin, Calcitonin, ACTH, GRH, Somatropin und viele andere verwandte Stoffe. Zur Resorptionsverbesserung sowohl über den Tränen-Nasen-Gang wie auch über die Comea, sofern eine topische Wirkung erwünscht ist, eignen sich eine Reihe von Hilfsstoffen wie z.B. Derivate der Cholsäure, Saponine, Glycerizin, Decamethonium und viele andere. Sofem eine unmittelbare Auflösung des Systems im Auge nicht erwünscht ist, können auch wasserunlösliche oder nur wasserquellende Polymere verwendet werden. In diesem Falle muss die folienförmige Arzneiform nach der Anwendungsdauer von vorzugsweise einigen Stunden bis Tagen aus dem Auge entfernt werden. Für dies e Sonderanwendung sind als wesentliche Polymere Polyvinylchlorid, Polyamide, Acrylester, Polyvinylacetate, wasserunlösliche Zelluloseester, quervemetztes Polyethylenoxid, quervemetztes Polyvinylporidon, quervemetzter Polyvinylalkohol und Polystyrol beispielhaft zu nennen.

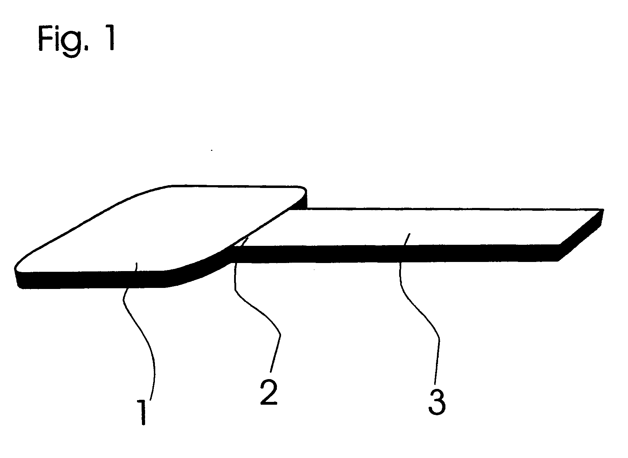

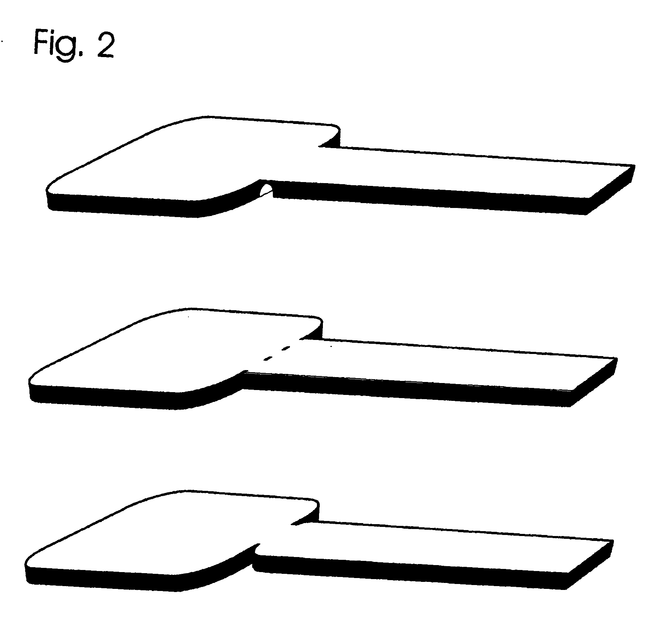

Der Applikator kann aus den gleichen Komponenten bestehen wie die folienförmige Arzneiform. Diese Konfiguration hat sicherlich herstellungstechnische Vorteile, ist aber für die erfindungsgemäße Funktion nicht zwingend. Vorzugsweise kann auch eine analoge Komposition unter Verzicht auf den Wirkstoffanteil erfolgen. Auch wenn für den Applikator eine stabile, stäbchenförmige Kontur aus Stabilitätsgründen vorteilhaft ist, kann es sinnvoll sein, zur Vermeidung von Verletzungen am Auge eine möglichst flächige, mit der folienförmigen Arzneiform konturähnliche Geometrie zu wählen. Eine mögliche Ausgestaltung ist beispielhaft in Figur 1 dargestellt. Der Applikator kann hier sinnvollerweise eine Länge zwischen etwa 1 und 10 cm aufweisen, eine Breite zwischen 2 und 10 mm sowie eine Dicke zwischen 20 und 500 µm. Die Sollbruchstelle zwischen Applikator und folienförmiger Arzneiform kann in einer unvollständigen Einstanzung, einer Verjüngung oder einer dicken Verminderung im betreffenden Bereich erfolgen. Die funktionale Auflösung der Sollbruchstelle am Applikationsort kann dem zur Folge durch Mechanismen der Auflösung wie auch des mechanischem Brechens der Kontur durch leichten Biegedruck erfolgen. Vorstehende Techniken sind in Figur 2 beispielhaft dargestellt, sollen aber den Umfang der Möglichkeiten nicht beschränken.

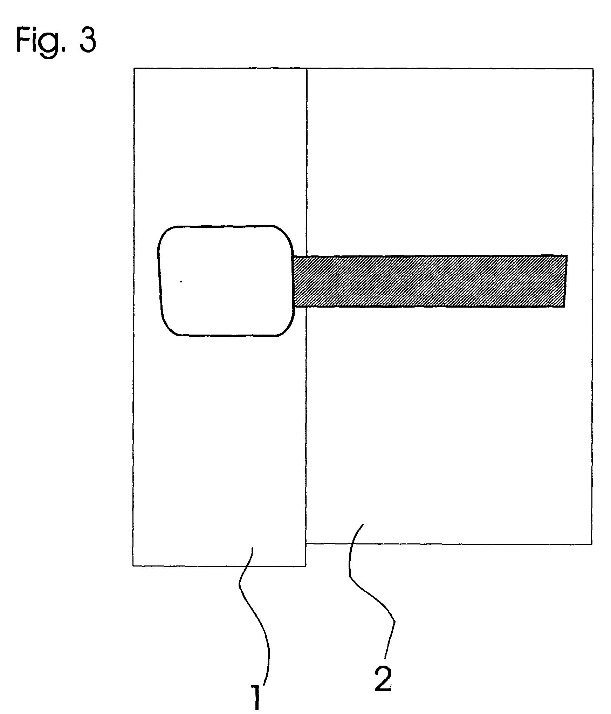

Die Herstellung der folienförmigen Arzneiform erfolgt durch Erzeugung einer Lösung oder Dispersion der Inhaltsstoffe unter Verwendung von Lösungsmitteln, insbesondere Wasser, durch anschließende Formgebung durch Extrusion oder Beschichtung und ggf. anschließendes Trocknen unter Anwendung von Wärme z.B. nach Verfahren wie sie im deutschen Patent P 4018247 genannt sind, auf das hiermit vollinhaltlich Bezug genommen wird. Eine besonders vorteilhafte Form der Herstellung ist die Fertigung von Applikator und folienförmiger Arzneiform in einem Arbeitsgang:
Die Beschichtung des arzneistoffhaltigen Bereiches (Figur 3: 1) und die Beschichtung des nicht wirkstoffhaltigen Bereiches (Figur 3: 2) erfolgt so, dass die folienförmige Arzneiform ausschließlich aus dem wirkstoffführenden Bereich 1 gewonnen wird, während der Applikator im Wesentlichen aus dem nicht wirkstoffhaltigen Bereich 2 gewonnen wird. Die Gewinnung der formgenauen Applikationseinheit erfolgt z.B. durch Aufstanzen mit geeigneten Werkzeugen (metallische Vollwerkzeuge, Bandstahlschnitt führende Werkzeuge etc.) oder aber durch andere Schneid- und Stanzverfahren nach dem Stand der Technik einschließlich z.B. der Möglichkeit des Laserschneidens gewonnen. Die Erzeugung der Sollbruchstelle erfolgt gleichfalls durch Verfahren der Stanzung, des Schneidens oder durch eine Konturierung der Beschichtungswerkzeuge in einer Form, dass in Höhe der Sollbruchstelle eine dünnere Beschichtungsqualität erzeugt wird.

### Beispiel:

20 g Polyvinylalkohol (Hydrolysierungsgrad 88%, Mowiol^{R} 8-88) werden in 80 ml Wasser unter Wärmeeinwirkung (bei ca. 80°C) und Rühren gelöst. Nach Abkühlung auf Raumtemperatur werden 0,2 g Natriumchlorid und 0,3 g Saccharose zugesetzt und gelöst. Die Lösung wird in zwei gleiche Anteile von je 50 g aufgeteilt (Phase 1 und Phase 2).
In 50,0 g Phase 1 werden 0,6g Naphazolin HCl aufgelöst. In 50,0 g Phase 2 werden 0,2 g Methylenblau aufgelöst.

20 g Phase 1 und 50 g Phase 2 werden nebeneinander derart in ein Filmbeschichtungsgerät (Spaltbreite ca. 100-120µm) gegeben, daß beim Beschichten auf polyethylenbeschichtetem Papier eine konfluierende parallele Beschichtung beider Phasen resultiert.
Der feuchte Film auf dem Papiersubstrat wird 10 Minuten bei Raumtemperatur und anschließend 10 Minuten bei 50°C getrocknet. Das Flächengewicht der getrockneten Schicht beider Phasen beträgt bei richtiger Spalteinstellung 20 g/m².

Der getrocknete Film wird anschließend mit einer die Kontur der endgültigen Applikationseinheit aufweisenden Schneidwerkzeug so gestanzt, daß der gesamte eingefärbte Teil im später für die Applikationseinrichtung vorgesehenen Bereich verbleibt. Durch Anwendung partiell schneidender Werkzeuge (erzeugt zum Beispiel aus Rasierklingenabschnitten) wird gleichzeitig oder anschließend die Sollbruchstelle eingestanzt.
Der folienförmige, wirkstoffhaltige Teil weist eine Fläche von 0,6 cm² (6 x 10 mm) bei 2 mg Eigengewicht auf. Der gleichfalls folienförmige Applikator erscheint bläulich und hat nach Stanzung die Maße 50 x 4 mm.

Die Applikationseinrichtung wird in peelfähige Siegelrandbeutel verpackt und zum Beispiell durch Gammasterilisation keimfrei gemacht.
Die Anwendung erfolgt am Auge zur Behandlung von Hyperämisierungen.

## Patentansprüche

1. Applikationseinheit von flächenförmigen wirkstoffhaltigen Darreichungsformen,
**dadurch gekennzeichnet, daß** diese Applikationseinheit aus einer folienförmigen Arzneiform selbst, einer Sollbruchstelle und einem Applikator besteht.

2. Applikationseinheit nach Anspruch 1,
**dadurch gekennzeichnet, daß** folienförmige Arzneiform eine Dicke von vorzugsweise zwischen 2 und 500 µm aufweist, in der Längsrichtung zwischen 3 und 20 mm, in der Breite zwischen 1 und 12 mm bei einem Gewicht zwischen 2 und 50 mg.

3. Applikationseinheit nach einem oder mehreren der voranstehenden Ansprüche,
**dadurch gekennzeichnet, daß** sie am Auge oder auf Schleimhäuten applizierbar ist durch Anfassen am Applikatorteil, Auflagen des Teils der folienförmigen Arzneiform auf den feuchten Applikationsort, Abwarten der durch Feuchteeinwirkung erfolgenden Abtrennung der Applikationsstelle und schließlich die Entfernung des Applikatorteils, der anschließend verworfen wird.

4. Applikationseinheit nach einem oder mehreren der voranstehenden Ansprüche,
**dadurch gekennzeichnet, daß** die enthaltene folienförmige Arzneiform pharmazeutische Wirkstoffe zur topischen oder systemischen Anwendung enthält, bevorzugt Peptide oder Nukleinsäurederivate mit therapeutischen Einzeldosen von unter 300 Mikrogramm.

5. Applikationseinheit nach einem oder mehreren der voranstehenden Ansprüche,
**dadurch gekennzeichnet, daß** die aufgebrachte folienförmige Arzneiform vollständig wasserlöslich ist.
